(19) Europäisches Patentamt
European Patent Office
Office européen des brevets

(11) Publication number: **0 003 670**
**A1**

(12) # EUROPEAN PATENT APPLICATION

(21) Application number: **79300182.7**

(22) Date of filing: **06.02.79**

(51) Int. Cl.[2]: **C 07 C 69/76**, C 07 C 67/30, C 07 C 79/46, C 07 C 101/453, C 07 C 149/43, C 07 D 317/46, C 07 D 317/54

(30) Priority: **06.02.78 AU 3280/78**

(43) Date of publication of application: **22.08.79 Bulletin 79/17**

(84) Designated Contracting States: **BE CH DE FR GB IT NL SE**

(71) Applicant: **COMMONWEALTH SCIENTIFIC AND INDUSTRIAL RESEARCH ORGANIZATION, Limestone Avenue, Campbell Australian Capital Territory (AU)**

(72) Inventor: **Holan, George, 86 Were Street, Brighton Victoria (AU)**
Inventor: **Walser, Reimund August, 11 Mitchell Road, Box Hill Victoria (AU)**

(74) Representative: **Lawrence, Peter Robin Broughton et al, Gill, Jennings & Every 53-64 Chancery Lane, London WC2A 1HN (GB)**

(54) **Preparation of alkyl 2-aryl acrylates.**

(57) Alkyl 2-arylacrylates are prepared by reacting formaldehyde with the appropriate arylacetic ester in a polar solvent in the presence of a base.

EP 0 003 670 A1

CSIRO
GJE/679/61

"PREPARATION OF ALKYL 2-ARYL ACRYLATES"

This invention relates to a method for the preparation of alkyl 2-arylacrylates.

In particular the invention is concerned with the preparation of alkyl 2-arylacrylates of the general formula I

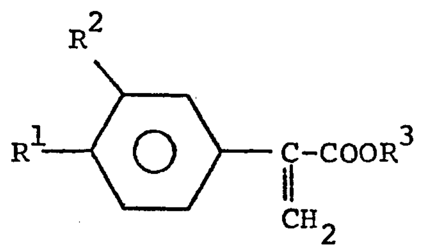

I

wherein

$R^1$ and $R^2$ are the same or different and each is hydrogen,
$C^1$ to $C^6$ alkyl, halo, $C_1$ to $C_6$ alkoxy or haloalkoxy, $C_1$ to $C_6$ alkylthio or haloalkylthio, nitro, or amino

or

$R^1$ and $R^2$ together form a methylene dioxy or halomethylene dioxy group;

or $R^1$ and $R^2$, together with the benzene ring, form a 2-naphthyl group;

and

$R^3$ is a $C_1$ to $C_6$ alkyl group. Any halogen is usually F, Cl or Br.

Some of the compounds of formula I have been described in our copending Australian patent applications Nos. 19829/76, PD 2818/77 and PD 6995/78, where they are described as intermediates in the production of insecticidally-active substituted 1-phenylcyclopropane carboxylates 1-phenylcyclobutane carboxylates and 1-(2-naphthyl)-cyclobutane carboxylates, respectively.

Furthermore, monomeric acrylates of the general formula I may be used, either alone or with other polymerizable monomers, to produce useful polymers. They can also be used as intermediates in the production of other chemical compounds.

Known methods of preparing alkyl 2-arylacrylates involve a three-step procedure, such as that described in our copending Application No. 19829/76, i.e., first condensing a phenylacetic ester with a di(lower alkyl) oxalate in the presence of an alkali metal alcoholate to produce an alkali metal enolate salt, then acidifying the salt to give the corresponding phenyl oxaloacetate, which is then reacted with formaldehyde under alkaline conditions to give the alkyl 2-phenylacrylate.

We have now found that conversion of an arylacetic ester to an alkyl 2-arylacrylate can be achieved in a single reaction step by reacting the starting ester with formaldehyde in the presence of a base in a suitable solvent.

Accordingly, the present invention provides a method for the preparation of compounds of the formula I as stated and defined above, which comprises reacting formaldehyde with a arylacetic ester of the general formula II

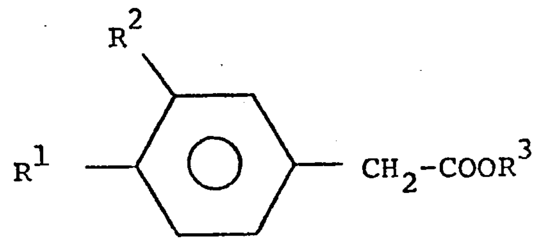

(II)

wherein $R^1$, $R^2$ and $R^3$ are as defined above, in a polar solvent in the presence of a base.

The base can be an alkali metal alcoholate, an alkali metal or alkaline earth metal oxide, hydroxide, carbonate, bicarbonate or salt of a weak organic acid, generally an aliphatic acid. Nitrogen bases, including aliphatic and aromatic amines, can also be used.

The solvent can be any suitable polar solvent, for example, dimethylformamide, dimethylsulphoxide, 1,2-dimethoxyethane, any ethylene glycol ether, or an aliphatic alcohol, e.g. ethanol, propanol or butanol.

Water can be present or absent and the formaldehyde can be supplied as paraformaldehyde or as a solution of formaldehyde in water.

The preferred solvent/base combination is dimethylformamide and potassium carbonate, which gives the best yields of arylacrylates when $R^1$ and $R^2$ are electron-donating groups. Amine bases and protonic solvents, such as aliphatic alcohols and monoethylene glycol ethers, are preferred when $R^1$ and $R^2$ are electron-withdrawing substituents.

The reaction may be carried out at ambient atmospheric pressure. The reaction temperature will depend on the nature of the substituents ($R^1$, $R^2$) on the aryl ring but will generally be between room temperature and about 80°C. The reaction is preferably conducted using, per mole ester, 1 to 5 (e.g. 2 to 4) moles formaldehyde and 0.1 to 5 (e.g. 0.1 to 1) moles base.

The product arylacrylates may be isolated by

normal work-up procedures or may be reacted further in situ without isolation.

The invention is further illustrated by the following examples.

General Method for the Preparation of Alkyl 2-Arylacrylates

The alkyl arylacetate (formula II) (0.1 mole) is dissolved in dimethylformamide (50 ml) and paraformaldehyde (0.2 mole), potassium carbonate (0.1 mole), and calcium oxide (0.1 mole) are added at once or in portions over 3 hours. The reaction temperature is kept at 40°C and the progress in the formation of the acrylate is followed by gas-liquid chromatography. When the starting material has disappeared (5-30 h) the reaction mixture is quenched in water and extracted with a solvent (ether or dichloromethane). On evaporation of the solvent, essentially pure acrylate is isolated. This can be further purified, if necessary, by chromatography through a short silica column.

EXAMPLE 1

Ethyl 2-(4-ethoxyphenyl)acrylate was prepared from ethyl (4-ethoxyphenyl)-acetate in 58.5% yield using the general method described above. The NMR and infrared spectra of the product were identical with those of an authentic sample prepared via the alkali metal enolate route (as described in our Australian patent application No. 19829/76, Example A).

EXAMPLE 2

Ethyl 2-(4-(α,α,β,β-tetrafluoroethoxy)phenyl)acrylate was prepared from ethyl 4-(α,α,β,β-tetrafluoroethoxy)phenylacetate in 37% yield using the general method. G.L.C.

analysis of the product gave a single peak. The NMR spectrum was consistent with the required structure:- δ ppm:1.3 (triplet 3H), 4.35 (quartet 2H), 5.95 (singlet, 1H - proton of the tetrafluoroethoxy group), 6.13 (doublet, 2H - $CH_2$ group of the acrylate), 7.3 (multiplet 4H - aromatic protons).

EXAMPLE 3

Ethyl 2-(4-nitrophenyl)acrylate

Ethyl 4-nitrophenylacetate (41.8 g) was slurried in 200 ml ethanol, 38.5% aqueous formaldehyde solution (31.2 ml) and sodium bicarbonate (1 g) were added and the reaction mixture stirred for 3 hours at 55°C. Most of the ethanol was removed by evaporation and the residue taken up in 50/50 water/diethyl ether suspension. After separation the ether layer was washed with water, dried over anhydrous sodium sulphate and evaporated to dryness to yield a faintly yellow viscous oil. This was purified by chromatography on silica gel eluting with dichloromethane to give ethyl 2-(4-nitrophenyl)acrylate in 10.5% yield.

EXAMPLE 4

Ethyl 2-(4-nitrophenyl)acrylate - Alternative Method

Paraformaldehyde was slurried in 150 ml of 1,2-dimethoxyethane, piperidine (5 g) was added and the mixture heated to 70°C. Ethyl (4-nitrophenyl) acetate (104.5 g) was added and the reaction continued for 20 hours. Most of the 1,2-dimethoxyethane was distilled off under reduced pressure and the residue was taken up in ether and filtered. The filtrate was evaporated to dryness and purified by chromatography on silica gel by eluting with dichloromethane to yield a yellow solid 74.6 g (67%)

after recrystallization.

EXAMPLE 5

Ethyl 2-(4-fluorophenyl) acrylate was prepared from ethyl (4-fluorophenyl)acetate by the general method in 51% yield. This compound was obtained in an impure state. However the NMR spectra showed the presence of a doublet $\delta = 5.95$ (2H) characteristic of the acrylate $CH_2$. The yield was confirmed by further conversion of the product to a difluorocyclopropane by reaction of the acrylate with a difluorocarbene and subsequent purification and identification of the difluorocyclopropane.

EXAMPLE 6

Ethyl 2-(4-butoxyphenyl) acrylate was prepared from ethyl 4-butoxyphenylacetate in 35% yield using the general method. The N.M.R. spectrum was consistent with the required structure: $\delta$ ppm 1.4 multiplet (10H) ($CH_2$ & $CH_3$ of butoxy and $CH_3$ of ethyl; 4.05 triplet (2H) (O-$CH_2$- of butoxy); 4.43 quartet (2H)($CH_2$ of ethyl); 6.05 doublet (2H)($CH_2$ of acrylate); 7.45 multiplet (4H) (aromatic protons).

EXAMPLE 7

Ethyl 2-(4-methoxyphenyl) acrylate was prepared from ethyl 4-methoxyphenylacetate in 37% yield using the general method. The N.M.R. spectrum was consistent with the required structure: $\delta$ ppm 1.35 quartet (3H) ($CH_3$ of ethyl);3.80 singlet (3H)($CH_3$ of methoxy); 4.2 quartet (2H)($CH_2$ of ethyl); 6.05 doublet (2H) (acrylate $CH_2$ ); 7.1 multiplet (4H)(Aromatic protons).

EXAMPLE 8

Ethyl 2-(3,4-methylenedioxyphenyl)acrylate was prepared from ethyl (3,4-methylenedioxyphenyl) acetate by the general method in 21% yield as a pale yellow oil. After removal of solvent and starting material the acrylate was not characterised as such but was converted to the corresponding dichlorocyclopropane by reaction with a dichlorocarbene. The N.M.R. and I.R. spectra of the ethyl 1-(3,4-methylenedioxyphenyl)-2,2,-dichlorocyclopropane carboxylate after purification were consistent with the required structure.

EXAMPLE 9

Ethyl 2-(2-naphthyl)acrylate was prepared from ethyl 2-naphthylacetate by the general method in 41% yield. The NMR spectrum of the product purified by chromatography showed the presence of a doublet $\delta = 6.13$ ppm (2H) characteristic of the acrylate $CH_2$. The structure of the product was further confirmed by conversion to ethyl 1-(2-naphthyl)-2,2-difluorocyclopropane carboxylate by reaction with a difluorocarbene. The 2-naphthyl difluorocyclopropane carboxylate was identical with an authentic sample.

CLAIMS:

1. A method for the preparation of alkyl 2-arylacrylates of the general formula I

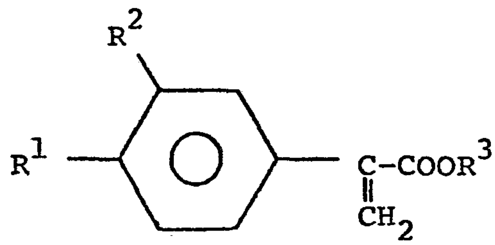

I

wherein

$R^1$ and $R^2$ are the same or different and each is hydrogen,
$C^1$ to $C^6$ alkyl, halo, $C_1$ to $C_6$ alkoxy or haloalkoxy,
$C_1$ to $C_6$ alkylthio or haloalkylthio, nitro, or amino;

or

$R^1$ and $R^2$ together form a methylene dioxy or halomethylene dioxy group;

or

$R^1$ and $R^2$, together with the benzene ring, form a 2-naphthyl group;

and

$R^3$ is a $C_1$ to $C_6$ alkyl group,

said method being characterised by the step of reacting formaldehyde with a phenylacetic ester of the general formula II

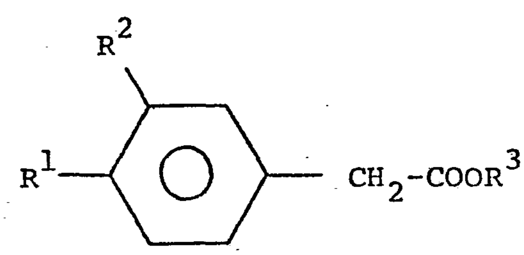

(II)

wherein $R^1$, $R^2$ and $R^3$ are as defined above, in a polar solvent in the presence of a base.

2. A method as claimed in Claim 1, characterised in that the base is an alcohol alkoxide, an alkali metal or alkaline earth metal oxide, hydroxide, carbonate, bicarbonate or salt of an organic acid or a nitrogen base.

3. A method as claimed in Claim 1 or Claim 2, characterised in that the solvent is dimethylformamide, dimethylsulphoxide, 1,2-dimethoxyethane, an ethylene glycol ether, or ethanol.

4. A method as claimed in any one of Claims 1 to 3, characterised in that the formaldehyde is supplied as paraformaldehyde or as a solution of formaldehyde in water.

5. A method as claimed in Claim 1, wherein one or both of $R^1$ and $R^2$ are electron-donating groups, characterised in that the solvent is dimethylformamide and the base is potassium carbonate.

6. A method as claimed in Claim 1, wherein one or both of the groups $R^1$ and $R^2$ are electron-withdrawing groups, characterised in that the solvent is a protonic solvent and the base is an amine.

European Patent Office

# EUROPEAN SEARCH REPORT

0003670
Application number
EP 79 30 0182

## DOCUMENTS CONSIDERED TO BE RELEVANT

| Category | Citation of document with indication, where appropriate, of relevant passages | Relevant to claim |
|---|---|---|
| X | DE - A - 2 013 641 (SYNTEX) * Page 14 upperpart of page; page 23, preparation 4 * ---- | 1-4 |

**CLASSIFICATION OF THE APPLICATION (Int. Cl.²)**

C 07 C 69/76
67/30
79/46
101/453
149/43
C 07 D 317/46
C 07 D 317/54

**TECHNICAL FIELDS SEARCHED (Int.Cl.²)**

C 07 C 69/76
67/30
76/46

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 16-05-1979 | KINZINGER |